(19) Europäisches Patentamt
European Patent Office
Office européen des brevets

(11) **EP 4 154 805 B1**

(12) **EUROPEAN PATENT SPECIFICATION**

(45) Date of publication and mention
of the grant of the patent:
**17.07.2024 Bulletin 2024/29**

(21) Application number: **22203161.9**

(22) Date of filing: **18.11.2010**

(51) International Patent Classification (IPC):
**A61B 5/0205** (2006.01)    **A61B 5/00** (2006.01)
**A61B 5/11** (2006.01)    **A61B 5/113** (2006.01)

(52) Cooperative Patent Classification (CPC):
**A61B 5/113; A61B 5/1102; A61B 5/7207;**
**A61B 5/725; A61B 5/7278;** A61B 2562/028

(54) **APPARATUS FOR MONITORING HEART RATE AND RESPIRATION**

VORRICHTUNG ZUR ÜBERWACHUNG DER HERZFREQUENZ UND ATMUNG

APPAREIL DE SURVEILLANCE DE LA FRÉQUENCE CARDIAQUE ET DE LA RESPIRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.11.2009 US 26233609 P
18.11.2009 US 26233109 P
24.08.2010 US 86188210**

(43) Date of publication of application:
**29.03.2023 Bulletin 2023/13**

(62) Document number(s) of the earlier application(s) in
accordance with Art. 76 EPC:
**10832160.5 / 2 501 277**

(73) Proprietor: **Texas Instruments Incorporated
Dallas TX 75243 (US)**

(72) Inventors:
• **PANDIA, Keya, R.
Dallas 75243 (US)**
• **RAVINDRAN, Sourabh
Dallas 75251 (US)**

• **COLE, Edwin, R.
Highland Park 75219 (US)**

(74) Representative: **Zeller, Andreas
Texas Instruments Deutschland GmbH
EMEA Patent Department
Haggertystraße 1
85356 Freising (DE)**

(56) References cited:
**US-A1- 2004 111 025    US-A1- 2005 240 087
US-A1- 2008 275 349    US-A1- 2009 105 556**

• **DAVID BARBOSA RENDON ET AL: "Mapping the
Human Body for Vibrations using an
Accelerometer", 2009 ANNUAL INTERNATIONAL
CONFERENCE OF THE IEEE ENGINEERING IN
MEDICINE AND BIOLOGY SOCIETY, 1 August
2007 (2007-08-01), pages 1671 - 1674,
XP055166546, ISSN: 1557-170X, DOI:
10.1109/IEMBS.2007.4352629**

**EP 4 154 805 B1**

# EP 4 154 805 B1

**Description**

This relates to apparatus for measuring, monitoring and analyzing biological and physiological functions of the human body, and to biomedical instrumentation and signal processing relating thereto.

BACKGROUND

Current apparatus and methods for monitoring human body functions, such as heart rate and respiration (e.g., monitoring belts worn about the chest, spirometers and canulas worn about the mouth and nose, and electrocardiogram (also referred to as ECG or EKG) electrodes and leads taped on the body) may be expensive, invasive or obtrusive and too cumbersome for ambulatory and continuous monitoring applications.

Noise mixed with signals received by the sensors used in heart monitoring, respiration monitoring, body motion and other monitoring applications can adversely affect the accuracy of each type of signal. Accordingly, methods for robust detection and separation of such signals in noisy conditions are desirable.

Conventional approaches to address the bodily motion signal separation and/or removal problem are believed to involve multi-signal adaptive algorithms that need an additional motion signal reference recording typically from a secondary sensor. Physical motion impulses from the feet couple very differently and in a non-stationary and non-correlated manner to sensors placed at different parts of the body and also to orthogonal axes of the same sensor. Accordingly, even using multiple sensors to cancel out an artifact may be complicated or unreliable.

In addition to medical-related applications, solving the above problems could also help monitor older adults for unexpected changes in gait, for falls, for syncope (fainting), for accidents and trauma incidents. Fitness monitoring at home, in exercise venues, and in institutional care settings could also benefit. In the European Search Report the following documents have been cited: DAVID BARBAROSA RENDON ET AL: "Mapping the Human Body for Vibrations using an Accelerometer" 2009 ANNUAL INTERNATIONAL CONFERENCE OF THE IEEE ENGINEERING IN MEDICINE AND BIOLOGY SOCIETY, XP055166546; US 2004/111025 Method and system using a non-electrical sensor for gating; US 2008/275349 Monitoring, predicting and treating clinical episodes; US 2009/105556 Measurement of physiological signals; US 2005/240087 Method and system for processing data from ambulatory physiological monitoring.

SUMMARY

The invention is defined by the appended claims.

Generally, and in one form of the invention, a respiration monitoring device includes a signal interface to receive and convert a signal input being an acceleration signal to an electronic form for further processing. An electronic circuit fed by the signal interface is operational to filter an acceleration signal in a manner to obtain a slow wander signal, to subtract from the acceleration signal the slow wander signal to obtain a heart sound signal, to derive a respiration signal from the heart sound signal, and to subtract the respiration signal from the slow wander signal to produce a substantially non-respiration body motion signal.

BRIEF DESCRIPTION OF THE DRAWINGS

FIG. 1 is a partially-block, partially-pictorial, partially graphical depiction of a structure and process for separating a respiration signal from heart and body motion and other signals using a single chest sensor.

FIG. 2 is a flow diagram of an inventive structure and an illustrative process for separating a heart signal from body motion and noise using a single accelerometer chest sensor and remarkable smoothing filter and residue generation, envelope-based noise rejection, and folded correlation.

FIG. 3 is a partially-flow, partially graphical depiction of a system and process separating a respiration signal, a heart signal and a body motion signal from each other using a single chest sensor.

FIG. 4 is a flow diagram of a system storage or process to inventively separate a respiration signal from a heart signal and using inter-beat intervals of the heart signal, with both the respiration signal and the heart signal substantially separated from body motion and noise signals.

FIG. 5 is a flow diagram of another system storage or process to inventively separate a respiration signal from a heart signal according to baseline wander method herein for respiration monitoring by a single inventively filtered accelerometer sensor.

FIG. 6 is a flow diagram of another system storage or process to inventively separate a respiration signal from a heart signal by amplitude modulation detection of peak heights of the heart signal.

FIG. 7 is a block diagram of an inventive wireless system structure and process and including inventive structures and processes from the other Figures.

## DETAILED DESCRIPTION OF EXAMPLE EMBODIMENTS

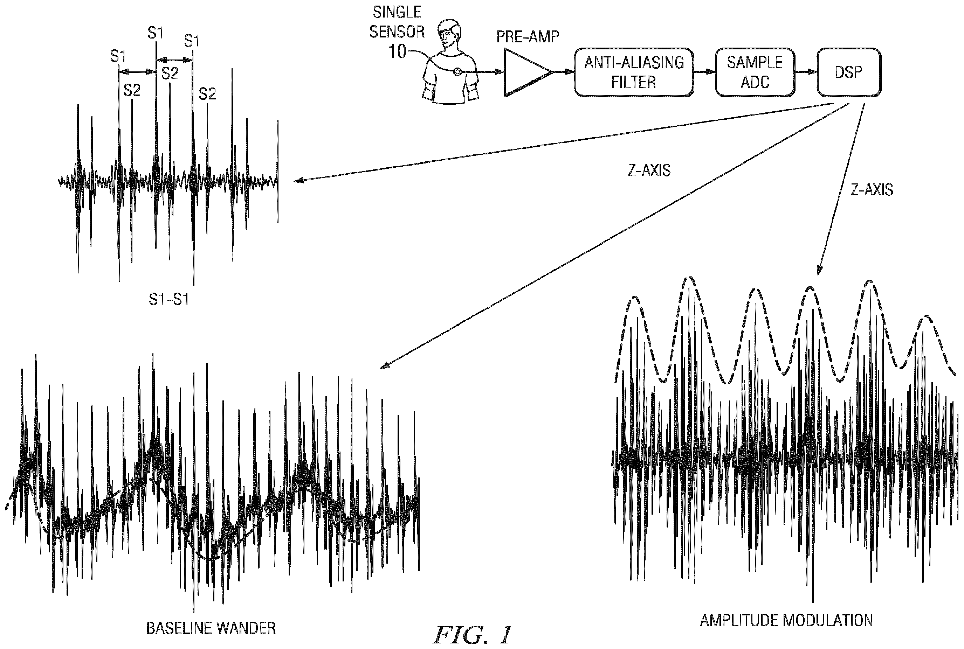

A miniature, high-sensitivity microelecromechanical system (MEMS) in a single, chest-worn sensor 10 herein records signals including some related to heart sounds. (The term "heart sound" refers in an expansive way to a signal analogous to cardiac S1, S2, and/or heart murmur or other cardiac waveform features, obtained from the processing of accelerometer data or other sensor data, and not necessarily to an audible sound.) A major challenge of ambulatory monitoring is the corruption of heart signals by body motion artifact signals and the confusion of such signals. In some measurements, the chest acceleration signal as picked up by the accelerometer 10 in FIG. 1 had a rather slow varying, but very strong (20-50 mv peak-to-peak) motion component. Riding on top of this motion signal was a higher frequency, but weaker (5-10 mv peak-to-peak), heart sound signal. Significant variability between subjects was observed in the frequency content of both the motion and the heart sounds. Also, signals for respiration, motion and heart sounds are not entirely frequency separable. Thus, more than simple digital band pass filtering is provided herein.

Example embodiments are described that separate and derive motion/activity, heart rate and respiration from a single signal derived from a single chest-worn sensor, such as a miniature Z-axis accelerometer sensor. Ambulatory measurement of respiration and cardiac activity can find wide application in home health monitoring of older adults and of patients with a history of cardiovascular, respiratory, and other conditions for which respiratory and/or cardiac monitoring are desired. Evaluating cardiovascular performance of patients in intensive care units (ICU) and hospital settings, in mobile ambulances, and at accident and trauma sites also calls for ambulatory cardiac and respiratory measurement and monitoring.

Various example embodiments use a single miniature, chest-worn MEMS accelerometer and associated monitoring circuitry to measure and monitor respiration, motion and heart activity -- reflected by heart sounds -- as shown in FIG. 1.

FIG. 1 illustrates example embodiments provided for ambulatory monitoring of heart rate and heart sounds, activity, body motion and respiration in a non-invasive and minimally obtrusive way. Here, a single sensor, such as with a MEMS accelerometer, extracts not only heart rate/heart sounds but also respiration in an ambulatory setting. Any signal is used that includes detectable heart sound signals from at least one sensor axis of the accelerometer sensor, or from two or more sensor axes. When a body motion signal component is included in the sensor signal, that body motion signal component is in some embodiments separated out or isolated and delivered as a useful output representing activity or motion as well.

Some advantages of various embodiments are extraction of three vitals (respiration, non-stationary bodily motion wander from activity, heart sounds/heart rate) from as few as a single sensor or signal capture component and a single signal. A miniature sensor embodiment taped on the chest provides a non-invasive and minimally obtrusive way to sense and monitor vital biomedical signals and physiological parameters in the presence of motion. Embodiments can be used with minimal inconvenience in ambulatory and continuous monitoring applications, and are very inexpensive and can be made into disposable patches and tapes, for instance.

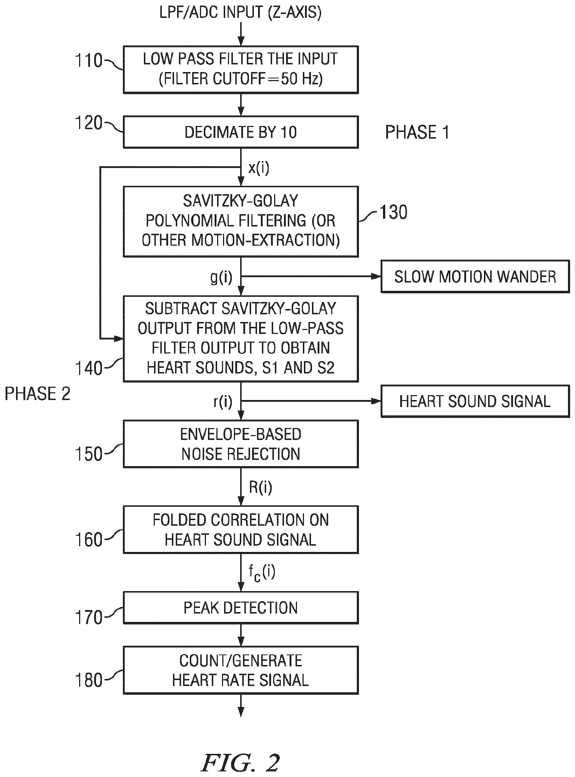

The device according to the invention includes a data acquisition/signal processing circuit having a special smoothing filter 130 (FIG. 2) that tracks slow varying body motion signal wander or variation, and then removes the wander from the sensor-based signal to give a clean (motion-removed) biomedical signal of interest on a display unit. The smoothing filter 130 involves a polynomial filter or comparably effective smoothing filter used directly or in a composite signal processing path. Some embodiments use a subtraction step 140 as in FIG. 2 to remove non-stationary motion artifacts reliably and robustly. Removing such artifacts makes the system more fully immune to sensor placement and contact variations on the chest that might arise when using sensor 10. This provides a simple, yet effective way to reduce the impact of motion artifacts and allow the reliable detection of primary heart sounds and subsequent derivation of heart rate even when a person is walking while being monitored. In this way, motion signal removal or separation, and heart-sound signal detection and heart rate detection are facilitated. No secondary reference or noise source is needed, thus reducing complexity of system design. Embodiments of structure and method thus extract primary heart sound signals from chest-worn sensor (e.g., accelerometer) data in the presence of motion artifacts.

In some structure and process embodiments for removal of motion-related artifacts from biomedical signals, beneficial monitoring is provided, e.g., for either or both of two independent signal sources -- accelerometer 10 and ECG if used. Chest acceleration signals are collected in an ambulatory (walking) setting from real human subjects using a chest-worn accelerometer 10 -- providing primary heart sounds signified as S1 and S2. Heart sound S1 includes audible sounds concurrent with tricuspid and mitral valve activity and shows on a seismocardiogram as a pulse bundle. Heart sound S2 is mainly associated with pulmonary valve and aortic valve activity. Structures and processes of the embodiments thus remove motion artifacts and facilitate the use of a single, miniature, chest-worn MEMS accelerometer to pick up heart activity and heart rate - derived from heart sounds -- from ambulatory subjects as shown in FIG. 1. For testing, electrocardiogram signals are independently collected from the human subjects walking briskly or running on a treadmill -- providing signal components such as QRS from the ECG.

Process and structure embodiments can also be extended to other biomedical signals corrupted by motion wander - e.g., electrocardiogram (ECG) , photoplethysmogram (PPG; signal from a pulse oximeter), electroencephalogram (EEG), electromyogram (EMG), impedance cardiogram (ICG) signals - or almost any other signal that might be affected by a separable wander. Thus, motion-related artifacts are removed from such other biomedical signals in products that can be produced by a manufacturer in volume.

A polynomial smoothing filter 130 (e.g., a Savitzky-Golay filter, such as implemented electronically) digitally smoothes a given accelerometer-based data signal stream by approximating it within a specified data window by a polynomial of a specified order that best matches the data in the window in a least-squares sense. Here, the electronic smoothing filter 130 fits the slower variations in body motion-induced components of the biomedical sensor-based signal and subtracts them as smoothed content from the biomedical sensor-based signal to leave behind what is called a residue signal. The residue signal provides a thus extracted, faster varying signal -- primarily the heart sounds and other cardiac activity, as well as some residual or remaining noise. Such polynomial filtering 130 preserves higher order moments around inflection points, or at extrema like peaks and troughs, that a digital moving average or low pass filter does not. In other words, the polynomial filtering better preserves features -- like local maxima and minima -- through a least-squares polynomial fit around each point.

In FIG. 1, a system embodiment has hardware that provides a measurement set-up and monitoring embodiment. A miniature (weight = 0.08 gram, size = 5x5x1.6 mm) triple axis, low-power, analog output MEMS accelerometer (e.g., STMicroelectronics Part No. LIS3L02AL) is taped onto the chest (e.g., a few inches to the left of the sternum along the third or fourth rib). (Taping the accelerometer sensor or using a chest band presses the accelerometer sensor to or against a bare or shaved portion of the chest and efficiently couples chest acceleration to the sensor.) An acceleration signal corresponding to the cardiac activity is captured along the Z-axis - the dorso-ventral direction orthogonal to the plane of the chest. The chest acceleration signal is AC-coupled with a 3 Hz cutoff and amplified with a gain of 100 and low pass filtered (for anti-aliasing) through a three-stage, five-pole Sallen-and-Key Butterworth filter with a 1kHz corner frequency. A commercial quad operational amplifier (op amp) package (e.g., Linear Technology Part No. LT1014CN) is used for the analog front-end. The accelerometer signal is then sampled at 10,000 Samples/sec using a data acquisition card (e.g., as available from National Instruments, Austin, Texas, USA) and captured and stored on a computer using MATLAB software (Version 2007b, The Mathworks, Natick, Massachusetts, USA). The AC coupling with approximately 3 Hz cutoff, which is a noncritical roll-off frequency, is provided, for example, by a series coupling capacitor C coupled to an input resistance established for the amplifier. For testing, a reference ECG (lead II) may be provided and acquired simultaneously in a three electrode (single lead) electrocardiogram ECG amplifier configuration as a standard of reference in order to compare with the accelerometer-derived cardiac signal for the evaluation of the performances of the heart rate extraction from the accelerometer signal.

In FIG. 2, for detection of primary heart sounds and cardiac activity, the acceleration signal is digitally low pass filtered in a step 110 at 50 Hz (e.g., using a 3326 tap digital FIR filter with a steep 80 dB roll-off over 20 Hz) and decimated in a step 120 by a factor of 10. (Roll-off frequency less than 60 Hz attenuates 60 cycle USA power line interference with biomedical signals of interest, and roll-off may be made less than 50 Hz for applicable countries using 50 Hz. While the roll-off frequency could be made higher, this FIR filter also desirably attenuates white noise above the frequency range of the signals being monitored.) Also in a Phase 1, a high order Savitzky-Golay (S-G) polynomial smoothing filter 130, using 28th order and 401 point frame, is used to capture the relatively slow-varying motion wander and leave out the more rapidly varying heart sound signal components. (Matlab syntax for such filter is g = sgolayfilt(X,28,401), where g is the filter output and X is a latest input column vector of 401 sample values of windowed data.) In a Phase 2, the smoothing filter 130 output is subtracted in a step 140 from the decimated LPF output to obtain heart sounds S1 and S2. A folded correlation process in a step 160 then enhances and strengthens the polynomial filtered S1/S2 peaks in the motion-removed acceleration signal. Then the location of the peaks is threshold-detected in a step 170 using an electronic amplitude-based peak picking process, and the selected peaks are counted in a step 180 to calculate heart rate HR.

Low pass filtering (LPF) below 50Hz is used in some of the examples because most of the desired signal power lies in that range and in general LPF with some roll-off frequency below about 100 Hz in many of the embodiments avoids making the bandwidth so wide as to encompass and integrate a substantial or undue amount of sensor noise (thermal, white spectrum). In case LPF with a roll-off frequency above power-line frequency is used, then some embodiments also include notch-filtering for power-line frequency rejection. Another embodiment extracts satisfactory S1-S2 heart signals from raw motion-affected accelerometer Z-axis data by low pass filtering with corner at 100 Hz and then Savitzky-Golay filtering at 20th order, followed by subtraction of the S-G signal from the LPF signal, and followed further by signal enhancement. It appears that polynomial filtering of motion-affected LPF accelerometer signals, using polynomial filtering on the order in a range of approximately 20th order or higher order to at least over 30th order, is satisfactory for obtaining heart signals as a residue by subtraction of the polynomial filtering output from the LPF signals. Using polynomial fits at such orders successfully captures both coarser and finer motion effects. The smoothing filter in some embodiments can be lower order as well, and may obtain good results even with a 1st-order polynomial in case of some window sizes and applications. Also, lower order polynomial filtering is contemplated and found useful as discussed later hereinbelow. Using a number of points at least approximately half again (1.5 or more times) an order of the polynomial and even substantially higher than that, in some of the embodiments, is believed to help to reduce noise.

The smoothing filter 130 of FIG. 2 is configured based on a specified order M and frame size (number of sample points N). For instance, a Savitzky-Golay polynomial smoothing filter is used in some embodiments to best approximate the acceleration signal in the least-squares sense to capture the motion-dependent baseline-wander. In some embodiments, the smoothing filter is implemented in flash memory of a local processor such as a belt-worn unit or provided in a home network gateway or clinic office network gateway, or cell phone or otherwise.

The matter of selecting and or finding feasible and optimum values for order M and window length (Nw in points, tw in time) for the polynomial smoothing filtering is discussed next. In general for a fixed window length, Nw, a higher order polynomial will fit the high frequency components of the streaming data better. For a given order M, a shorter window of time will allow fitting the high frequency component better.

A way to approach the optimization problem estimates the inherent order of the low-frequency component and picks the smallest window that satisfies the condition that Nw > M+1 and Nw is odd (i.e., Nw = 2N+1). The smaller the window size Nw, the smaller will be the number of taps of the multiply-accumulate filter process implementing the smoothing filtering. For an accelerometer signal in some applications, order M=1 and window size Nw =3 (sampling frequency is 1000 Hz). In some examples herein, higher orders M and window widths Nw are shown.

In FIG. 2, motion, heart sounds and heart rate are electronically separated and ascertained from accelerometer 10 data using the following steps: a) low pass filtering 110 and decimating 120 the accelerometer data; b) Savitzky-Golay filtering 130 to fit the relatively lower frequency motion data; c) subtracting 140 the output of the Savitzky-Golay filter from the low pass filtered accelerometer data (from step a) to obtain the heart sounds; d) performing 160 folded correlation to enhance the primary heart sounds (S1 and S2) peak locations; and e) peak picking 170 to count the number of S1 peaks in a predetermined or configured segment (time interval) and counting 180 the heart rate (HR) in beats per minute (BPM).

Note that the term 'decimation' refers to any process of regularly removing samples from a sample stream, or passing one sample in every no samples as decimation parameter, and can (but does not necessarily) refer to removing all but one sample in ten. Thus, if a sample/ADC delivers fs samples per second, then a decimation process delivers a decimation frequency substantially $f_S/n_D$ samples per second. If a window period is tw seconds, then the number of points Nw = 2N+1 in the window is Nw = $1+f_S t_W / n_D$. The window period tw may be selected by considering the time period over which the particular features and behavior of interest are to be obtained by the filtering from the signal. The sampling frequency fs may be selected with cost, physical size and complexity of anti-aliasing in mind (low pass filter AAF at 0.5fs or less situated ahead of sampling fs). The sampling frequency fs may be set substantially greater than the Nyquist frequency for sampling the AAF (anti-alias filter) output. The decimation parameter $n_D$ is then selected, firstly, to yield a decimation frequency $f_S/n_D$ that is sufficiently high relative to the, e.g., 50 Hz low pass filter LPF following the sampling/ADC circuit to provide effective operation of that LPF. Secondly, the decimation parameter $n_D$ is also selected to yield a number Nw of window points that is sufficiently high relative to the selected order M of the filter to keep filter noise low while having the Nw window points being sufficiently low in number as to introduce only so many filter computations as needed to achieve satisfactory filtering of the signal stream in the window. The filter computations are related to the product of the number Nw of points per window multiplied by a rate number rw of windows processed per second. If rw = Nw/ tw, the computations are proportional to $N_W^2 / t_W$, which may motivate fewer window points and longer window times in some energy-saving and lower cost processor applications.

Mathematically expressed electronic processes are described in further detail below for preparing various electronic embodiments with smoothing filters for various ways of motion extraction in step 130 and any other purpose to which their advantages commend their use. They are appropriately partitioned into offline and real-time online electronic processes in such embodiments. The notation $\|(x-Ab)\|$ in Equation (1) below signifies the sum of squared differences between the [(2N+1)×1] respective data stream vector sample points or stream components and the (2N+1) respective estimates of those stream components provided by multiplying a [(2N+1)xM] transform matrix A times a [M×1] vector of transform coefficients $b_j$. The number of transform coefficients $b_j$ is M, and they form a [M×1] vector b. A gradient∇ is the [M×1] vector of first partial derivatives with respect to the transform coefficients $b_j$. The number M of coefficients $b_j$ is called the order, and if the number of transform coefficients $b_j$ is M, then the order of the process is M. The [MxM] matrix of second partial derivatives with respect to the transform coefficients $b_j$ is signified by $\nabla \nabla$. The filter procedure involves, and in effect forms, a coefficients change coefficient vector $\Delta$ b for updating an initial transform coefficient estimate b=0 (i.e., all coefficients initialized to zero). This procedure pre-multiplies the matrix of second partial derivatives times the negative of the gradient to obtain that transform coefficients change vector:

$$\Delta b = - (\nabla \nabla \|(X\text{-}Ab)\| )^{-1} \nabla \|(X\text{-}Ab)\| . \qquad (1)$$

Since the Equation (1) involves a quadratic expression and starts from b=0, the process directly finds the values of the transform coefficients b = $\Delta$b in one pass without iterating additionally.

Equation (2) represents the result of performing the calculus operations represented by Equation (1). (Some embodiments transmit the coefficients b from Equation (2) to a remote site for record storage and further analysis, since they effectively compress much of the information in the data window. If coefficients are to be transmitted, the [Mx(2N+1)] matrix $(A^{T}A)^{-1} A^{T}$ is pre-computed and then multiplied by each data window locally on the fly. Other embodiments omit such compression and/or transmission, or only do it locally on remote command, and thereby save some power and processing complexity.)

$$b = (A^{T}A)^{-1} A^{T} X , \qquad (2)$$

provided the inverse $(A^{T}A)^{-1}$ exists -- when the rows of the matrix A are linearly independent (full rank) and enough data points Nw = (2N+1) deliver enough corresponding columns of the matrix sufficient for an inverse to be delivered.

In the special case of a polynomial transform process, a matrix of indices is raised to powers, wherein the jth column element $A_{nj}$ in the nth row of transform matrix A is raised to a power: $n^{j}$. In other words, for the 2N+1 different values of n from -N to +N in the window of a data stream X(i+n), the transform finds a set of coefficients $b_{j}$ for a well-fitting power series to approximate all the values. Such a power series in general is

$$X'(i+n) = b_{o} + b_{1} n + b_{2} n^{2} + b_{3} n^{3} + b_{4} n^{4} + ... b_{M} n^{M} . \qquad (3)$$

Savitzky-Golay filtering outputs as the filter output g(i) for the window indexed i the value of $b_{0}$ estimated by Equation (2) for each data window, and successively window-by-window for successive indices g(i). Rows of matrix A are orthogonal when the inner product is zero for any pair of different ones of them. These rows are illustrated in Table 1, below. The rows of values $A_{nj}$ in matrix row n are non-orthogonal for the example of a polynomial transform. ("^" signifies raising to a power) .

Table 1: Arrangement of Matrix $A^{T}$

| Points | 0 | 1 | 2 | 3 | ... M |
|---|---|---|---|---|---|
| n=-N: | [1 | (-N) | (-N)^2 | (-N)^3 | ...(-N)^M]. |
| ... | | | | | |
| n=-1: | [1 | -1 | 1 | -1 | ... (-1)^M] |
| n=0: | [1 | 0 | 0 | 0 | ... 0^M] |
| n=1: | [1 | 1 | 1 | 1 | ... 1^M] |
| n=+N: | [1 | N | N^2 | N^3 | ... N^M]. |

Power m (Order M)

Next, the process finds an estimated data stream

$$X'=Ab = A (A^{T}A)^{-1} A^{T} X . \qquad (4)$$

An electronic process is set up in a processing circuit as represented by Equation (2) and electronically executed by the processing circuit. For Savitzky-Golay filtering, the process is optimized to only find g(i) as the estimated value of $b_{0}$ and also to perform as much off-line pre-computation as possible. Accordingly, Equation (4) is revised as in Equation (5) below, to use only the n=0 row [1xM] of the first pre-multiplying matrix A instead of the whole matrix A in Equation (4),

$$g(i) = [1 \ 0 \ 0 \ ...0] (A^{T}A)^{-1} A^{T} X(i) . \qquad (5)$$

Sometimes a mathematical presentation of Savitzky-Golay filtering regards the window as multiply-added by a set of (2N+1) filter coefficients c(n). Here, a [1×(2N+1)] filter coefficient vector C is introduced so that

$$g(i) = C\, X(i) = \sum_{n=-N}^{+N} c(n)x(i+n), \qquad (6)$$

where

$$C = [1\ 0\ 0\ \dots 0]\,(A^T A)^{-1}\, A^T. \qquad (7)$$

In Equation (8), an alternative notation CI equivalent to Equation (6) post-multiplies Equation (7) by a $[(2N+1)\times(2N+1)]$ identity matrix I and designates each of the columns of that identity matrix I as $[(2N+1)\times 1]$ unit vectors $\varepsilon_n$. The phrase 'unit vector' for $\varepsilon_n$ means a $[(2N+1)\times 1]$ vector of all zeroes except for a one (1) at the nth row position. Furthermore, only the matrix inversion computations to form the first row of inverse matrix $(A^T A)^{-1}$ are relevant and are performed, considering the pre-multiplication by $[1 \times M]$ row n=0 vector $[1\ 0\ 0...0]$. Thus, the filter coefficients are also equivalently expressed in the notation of Equation (8), which is equivalent to Equation (7).

$$c(n) = \{(A^T A)^{-1}\,(A^T \varepsilon_n)\}_0. \qquad (8)$$

The Savitzky-Golay filter does a local polynomial fit in a least square sense. For a given input variable data window $x(i+n)$ and window of length $2N+1$ and chosen polynomial degree M, the filter output is given by $g(i)$. Filter coefficients $c(n)$-2N+1 of them-are computed, e.g. off-line, by electronic operations represented by Equation (7) or (8) and loaded into flash memory of a small signal processing unit either worn on the person or provided nearby and coupled wirelessly to the accelerometer sensor 10. The signal processing unit suitably has a digital signal processor circuit such as processor 220 that electronically performs multiply-accumulates (MACs) represented by Equation (6) according to a stored program accessing the filter coefficients $c(n)$.

Some other embodiments use windows that are not centered around the value at index n used as the output (e.g., n=0). A variety of choices of matrices A are possible instead of the particular polynomial transform matrix shown in Table 1. The skilled worker chooses the desired transform, the window (frame) size (e.g., 2N+1) and the order M. Also, note that $g(i)$ output of a first filter procedure produces a data stream that itself can be windowed as represented by column vector $g_1(i_2)$ in Equation (9B). Accordingly, some embodiments represented by Equations (9A), (9B) cascade two lower order filters of Equation (4) and use straightforward technique to minimize the electronic processing complexity of the computations in implementation. The transform matrices A1 and A2 can be the same or different, the window sizes $(2N_1+1)$ and $(2N_2+1)$ can be the same or different, and the orders $M_1$ and $M_2$ can be the same or different, all these choices being independent of each other.

$$g_1(i_1) = [1\ 0\ 0\ \dots 0]\,(A_1^T A_1)^{-1}\, A_1^T\, X(i_1),\ \text{and} \qquad (9A)$$

$$g_2(i_2) = [1\ 0\ 0\ \dots 0]\,(A_2^T A_2)^{-1}\, A_2^T\, g_1(i_2). \qquad (9B)$$

Some embodiments may also apply to the SG process a diagonal weighting matrix W which is all zeroes in a $[(2N+1)\times(2N+1)]$ matrix except for weights down the main diagonal. The weights can, for instance, be one at the middle of the diagonal and diminish symmetrically in value farther from the middle of the diagonal. Equation (1) is replaced by:

$$\Delta b = -(\nabla\nabla\|W(X-Ab)\|)^{-1}\ \nabla\ \|W(X-Ab)\|. \qquad (10)$$

Then the electronic process represented by Equation (5) for the output instead is:

$$g(i) = [1\ 0\ 0\ \dots 0]\,(A^T W A)^{-1}\, A^T W\, X(i). \qquad (11)$$

The selection of transform type and matrix A is fixed/predefined by configuration or determined semi-static manner in some embodiments. Dynamic configuration or selection of the matrix A or transform type or parameters of a given transform is contemplated.

Some other embodiments store and average a set of values from the transform output of Equation (4) from different windowed segments of the data stream X. This approach, roughly speaking, performs several filters in parallel and averages them in an offset manner. All the values represent a reconstructed value corresponding to a same instant of time (i+n) = t, and note that this approach not only uses the n=0 row to approximate $b_0$ but also uses the transform approximations to the other coefficients that are available from Equation (3). The electronic processor 220 (and/or 240) performs the electronic process as represented by Equation (12), where X'(i+n) is from Equation (4).

$$g(t) = [1/(2N_1+1)] \sum_{n=-N}^{+N} X'(i+n)|_{(i+n\,=\,t)} \quad . \qquad (12)$$

In view of the analysis herein, it is emphasized that other types of processes can be alternatively selected according to the teachings herein, whether they are called Savitzky-Golay or not. The skilled worker sets up a test bench with library accelerometer-based waveforms and then makes the transform matrix choices, choice of number of points (2N+1), and choice of order-value M, either manually or by an automated process. The filtering choices are tested either by visual inspection of a display of output from FIG. 1 process or by automated process according to metrics of false negatives and false positives, etc. as described herein. Transform matrix A values, and values of (2N+1) and M, $2N_1+1$, etc. for one or more such filter processes having favorable metrics are then loaded into the monitoring device flash memory or hard drive and executed in real time on processor 220 (or 240).

A transform for an embodiment approximates an actual data stream vector x(i+n) and produces an output signal stream that follows the heart sound peaks well over time in response to a data stream X herein derived from a body-worn accelerometer. Some embodiments have reduced processing complexity by using low enough frame size (2N+1), order M and/or using an efficient transform matrix A to achieve desired performance for the purposes for which the monitoring is intended. The same transform is desirably low-complexity and well-performing over numerous patients, accelerometers and their positioning on the body, and in different environments of use, such as clinic, hospital, home, exercise venue, etc.

In envelope-based noise rejection 150, an amplitude envelope is generated. The residue signal stream r(i) = x(i)-g(i) of of FIG. 2 has some remaining noise from subtracting step 140 that subtracts the smoothing filter output g(i) from the LPF-supplied input x(i). An envelope is fitted to the residue as in FIG. 1. The amplitude-based processed output R(i) is derived from the envelope-fitted residue r(i). The noise n(i) near the horizontal axis is substantially reduced. Operations for this process suitably use an envelope-related variable gain function. Alternatively, the circuit and/or process is arranged to generate zero signal output when the envelope is below a low threshold that still passes the peaks.

In folded correlation 160, an input data stream of residue r(i) = x(i)-g(i) , or envelope-processed residue R(i) comes to the folded correlation process. Recall that g(i) is the output of the smoothing filter such as represented by Equation (6) or from a buffer memory for it. Processed filtered residue R(i) is windowed by a further data window (also called a frame) of length $2N_2+1$ (with points accessed by an index n=0,1,2... $N_2$).

The output $f_c(i)$ of the folded correlation is given by Equation (13):

$$f_c(i) = \sum_{n=0}^{N2} R(i - N_2 + n)\, R(i + N_2 - n) \; . \qquad (13)$$

The digital data stream for heart monitoring residue signal samples R(i) from the smoothing filter subtraction is successively processed in overlapping frames indexed i. In general, the value of $2N_2 + 1$ is selected to be approximately the width tw of a desired signal event (e.g., an S1, S2, or R-wave). For example, S1 is typically about 100-150 milliseconds long. If the decimated sampling frequency fs is 1000 Samples/sec, the value of $2N_2 + 1$ is established, e.g., as an odd number between 101 and 151, and N2 is some number between 50 and 75 inclusive. Thus,

$$N2 = RND(tw\, fs\, /2). \qquad (14)$$

The Folded Correlation 160 in another example has a frame size of 7 at with the 1000 samples/sec that resulted from decimation. The peaks corresponding to S1 and S2 in the motion-removed acceleration signal are thus strengthened and are then peak-detected in step 170 for counting 180. In some embodiments, the value of N2 is configured in flash memory, and can be selected or altered by a local or remote operator. In the electronic folded correlation process 160 represented by Equation (13), the heart monitoring residue samples R(i + N2 - n) from the later half of each frame are folded around the center heart monitoring sample R(i) in the frame and multiplied by dot product (sum of products in Eq. (13)) with heart monitoring residue samples R(i - N2 + n) in the earlier half of each frame. The result of the dot product is a folded correlation output signal stream $f_c(i)$ corresponding to instant i of the input residue signal stream R(i) in the center of the frame. Succeeding thresholding passes the S1 peaks and counts them. Robust detection of primary heart sounds and heart rate from a chest-worn accelerometer is thus achieved in the presence of interfering motion artifacts.

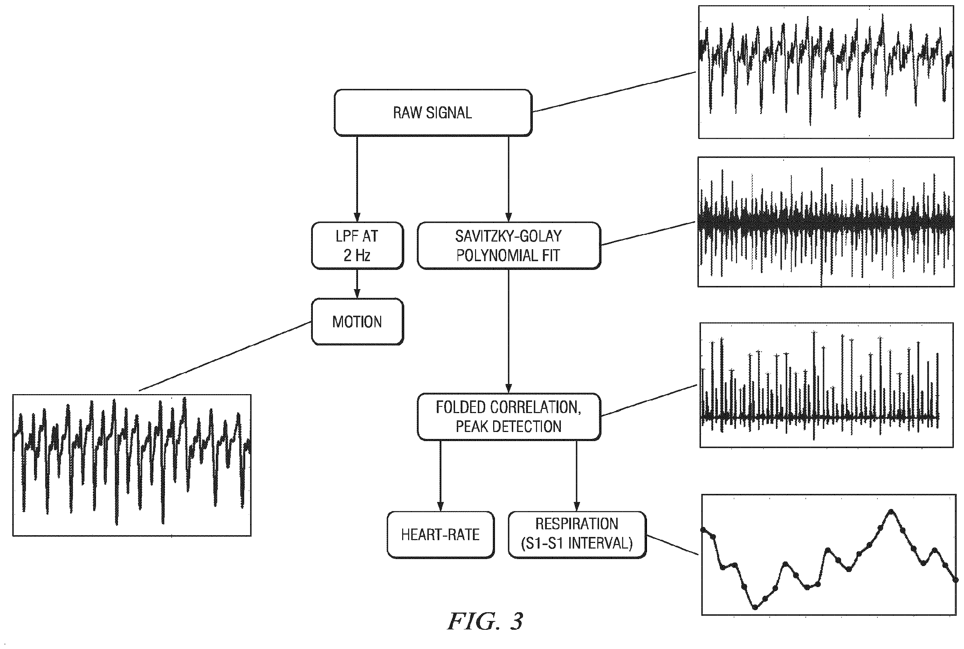

In FIG. 3, a respiration waveform is obtained, for example, by a monitoring device embodiment of FIG. 1 and its corresponding process, the latter not being part of the claimed invention. The process in FIG. 3 receives the raw signal stream from the analog-to-digital converter (ADC) of FIG. 1 and then first separates the heart sounds from the composite signal from the sensor using Savitzky-Golay (S-G) polynomial fitting, followed by folded correlation and peak detection to deliver the S1 heart signal peaks. The heart rate is counted in response to the peak detection to provide a heart rate signal output. Concurrently, the respiration is monitored by then measuring the successive times, called the inter-beat intervals or S1-S1 intervals, between heart beats - beat by beat. The variation in the measured inter-beat interval over time thus represents respiration because it is respiration-dependent and substantially independent of non-respiratory gross body motion. The monitoring device thus delivers as a respiration waveform that substantially represents the inter-beat interval varying over time. Further respiration processing counts the breathing rate and delivers a resulting breathing rate output, and derives and outputs any other useful information.

Detection of respiration from the inter-beat interval may involve a phenomenon called respiratory sinus arrhythmia RSA, which is possibly responsive to respiration-related and other intrapleural or intra-thoracic pressure changes. In the meantime, the motion signal is extracted either from the S-G polynomial smoothing filter 130 as in FIG. 2 or by a low pass filter LPF, such as a digital finite impulse response (FIR) filter with corner frequency at 2Hz as shown in FIG. 3.

In FIG. 3, post-processing of the motion signals is applied to monitor and deliver waveforms representing average activity level over time, monitor walking gait and other motions, and detect a fall if one were to occur. Consequently, deriving motion or activity from an accelerometer is important, such as by the present embodiments. For instance, average activity level can be generated as the root-mean-square (RMS) of the motion waveform measured over an hour and output hour by hour. Walking gait can be derived from the Z-axis alone or in combination with signal streams from other accelerometer axes with respiration subtracted out. A fall sensor embodiment includes a peak detection of an unusually high-magnitude acceleration peak which stands out from any recent or subsequent neighbor peaks in a predetermined window of time such as +/-15 seconds.

Exemplary processes illustrating the present invention as in FIGS. 1 and 3 separate motion signal components from an accelerometer sensor signal to cleanly and robustly extract heart sounds and also respiration (derived from heart sounds) in the presence of motion and activity, and further to deliver a motion/activity signal. In some embodiments, motion-based gating is performed to reject signal frames in the event that the motion/activity level is unusually high and does not permit reliable detection of heart rate or respiration or some other derived signal under a given detection process. The monitoring device measures gross body motion such as to sense and monitor activity and can provide a useful index of a person's level of activity over a period of time, and facilitate inferences about the person's lifestyle and metabolic index -- jointly with ECG-derived heart rate. Gait recognition by accelerometer-based motion monitor aids biometric assessment and can identify signs of or precursors to a dangerous fall. Also, using the accelerometer-based motion monitor as an indicator of sudden, high-magnitude acceleration can additionally identify signs of or precursors to a dangerous fall, as well as a fall itself.

In FIG. 3, the signal obtained during motion from the chest-worn accelerometer is digitally low pass filtered at 2 Hz (using a digital FIR filter) to extract the slowly varying baseline wander due to motion. For at least some cases of body motion as well as the body at rest, the respiration signal is well decoupled in frequency from the low-frequency body motion signal and is thus digitally low pass filtered to successfully extract the respiration signal.

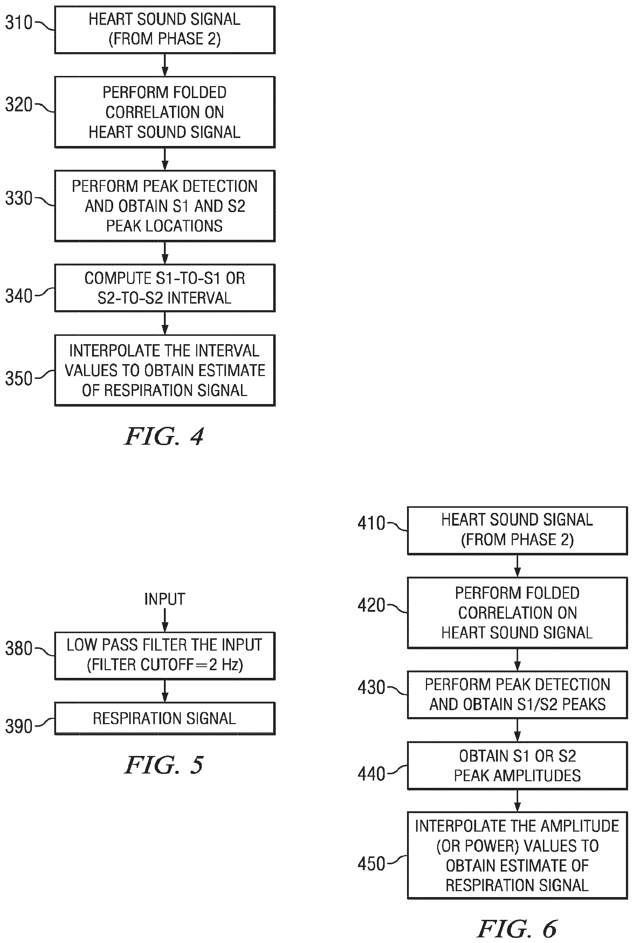

FIGS. 4, 5, and 6 respectively show three exemplary processes or methods of electronically generating respiration signal outputs from a sensor input. These processes or methods are not covered by the claimed invention. In various embodiments, these processes are used either individually, or in pairs, or a combination of all. Some embodiments as illustrated in FIGS. 1, 3 and 4 derive respiration from an accelerometer sensed-and-residue-detected variation in the S1-S1 interval during motion.

In FIG. 4, a process obtains the heart sound signal at a step 310 by removal of motion-dependent wander in Phase 2 of FIG. 2. The heart sound peaks are reinforced through Folded Correlation 320, and peak detection 330 is performed to detect the S1-S1 peaks. In a step 340, the S1-to-S1 interval (or S2-to-S2 interval) is repeatedly computed beat-by-beat to electronically obtain data values of successive inter-beat intervals. These data points are interpolated in a step 350 to yield a continuous respiration waveform of FIG. 3.

In FIG. 5, another process is called the baseline wander method herein for respiration monitoring by a single accelerometer sensor. This process in a step 380 operates on the raw accelerometer ADC signal input from a person at rest by low pass filtering it with a filter cutoff frequency at about 2 Hz, or otherwise selected, e.g., with LPF cutoff in a range of about 1 Hz to about 3Hz. A waveform called 'baseline wander' thus obtained as an electronic respiration signal at a step 390. In other words, breathing periods of about half a second or more are passed, so that not only resting breathing periods of a breath every two or three seconds are detected, but also breathing periods under stress or after exercise down to about a third or half a second are detected. Shorter period signal variations in the accelerometer are suitably obtained from the S-G filter smoothing or by other types of filtering to represent body motions other than respiration.

In FIG. 6, a further for respiration monitoring by a single accelerometer sensor 10 obtains the heart sound signal at a step 410 by removal of motion-dependent wander in Phase 2 of FIG. 1. Folded correlation 420 reinforces heart sound peaks, and peak detection 430 detects the amplitude modulated S1 peaks. Instead of (or in some embodiments in addition to) inter-beat interval measurement as in FIG. 4, the successive S1 peak amplitudes (or S2 peak amplitudes) in a step 440 are repeatedly measured electronically beat-by-beat in FIG. 6 to electronically obtain data values of successive heart sound peak amplitudes. These data values are interpolated in a step 450, such as by linear or quadratic or other interpolation, to yield a continuous respiration waveform.

Various respiration parameters are obtained by finding various values on the respiration waveforms and differences and trends therein. Respiration rate is measured as the number of cycles of inhalation and exhalation in a given time window (e.g., one minute). Averaging and signal fusion methods/algorithms robustly derive respiration rates from multiple parameters. How deeply the person is breathing is represented by an average of the difference between successive values of inhalation peak and exhalation trough in a given time window (e.g, one minute). Averages and trends in the inhalation peaks and/or exhalation troughs are readily calculated and displayed respectively.

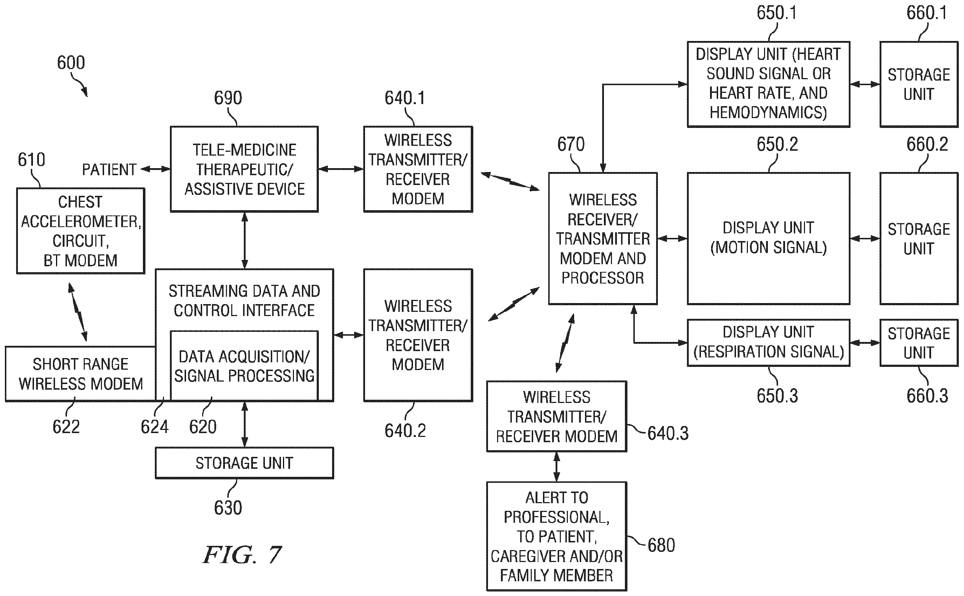

FIG. 7 shows a wireless system embodiment 600 for a respiration and cardiac monitoring system including various remarkable device or component embodiments. Some embodiments are wired embodiments, and FIG. 7 shows a wireless embodiment. An accelerometer sensor 610 and its electronic circuit also have a Bluetooth or other short range wireless modem wirelessly coupled to another short range wireless modem 622 that is coupled via a streaming data and control interface 624 to a data acquisition signal processing unit 620. Further, modems 640.2 and 670 for wireless transmission and reception remotely are provided at each of two locations so that the data acquisition signal processing unit 620 communicates via its modem 640.2 to the remote wireless transceiver unit or modem 670. The latter modem 670 is coupled to be one or more display units 650.i and their storage unit(s) 660.i. In this way, telemedicine applications are supported. The acquisition signal processing unit 620 and its modem 640.2 are suitably provided in a residence or ambulance or on the person or in a wheelchair or gurney. The wireless transceiver 670 and display unit(s) 650.i are suitably provided in a clinic, hospital, medical monitoring center or otherwise. Either or both ends of the wireless system may be mobile, such as one example of a modem 640.3 and alert/processor/display 680 when a professional in a vehicle is urgently needed to review data coming in from a residence or another vehicle in case of emergency and to respond with instructions.

In FIG. 7, combinations with further processes, circuits and devices for automatic cautionary responses, warnings, and/or automated monitored therapeutic responses are contemplated. Upon occurrence of undue excursions of one or more measured parameters or relationships among parameters detected by signal processing unit 620, the remote processor 670 alerts any one or more of medical professional, patient, caregiver, and/or family member via a modem 640.3 and alert/processor/display unit 680 by sending a cellular telephone call and/or other voice call or message and/or written alert such as an automatic e-mail. The alert system suitably provides for acknowledgement by any of the recipients. Also, another modem unit 640.1 is suitably provided and coupled to a telemedicine therapeutic or assistive device 690 for assisting the patient in some pharmacological, informational, or physically assistive way by administering a medicine, or adjusting a dosage or otherwise. In case of excursions that indicate an extreme medical emergency, the data acquisition signal processing unit 620 may be permitted to locally control the therapeutic or assistive device 690 temporarily and in a maximally-safe way until remote commands are received or first responders can arrive. Mere removal or inadvertent detachment of the accelerometer 610 from the chest is distinguished by the electronic processing 620 from affirmatively detected excursions of measured signals and parameters. Regarding telecommunicated care (telecare) assistance, such assistance is suitably rendered in some physical way in response to the real-time accelerometer sensor 620 data by activating motorized apparatus comprehended by device 690 such as to adjust a motorized bed, or move a scooter into proximity for patient use, or servomechanically actuate and flush a toilet unit, or open a tub drain to empty a tub, or some other assistance.

Types of embodiment, among others, can form: 1) a patient-worn medical-sensor and/or therapeutic device that is wired or has a wireless modem; or 2) a patient-worn signal processing and modem module on a belt clip that connects or wirelessly couples to such a sensor and wirelessly couples to a premises gateway or directly transmits to a remote location; or 3) a sensor, signal processor, memory, and modem together in a micro-miniature device that is taped to the chest and communicates to a router or gateway locally, and the latter communicates remotely; or 4) a local router or gateway that includes signal processor, memory, and multiple types of modem to communicate with the sensor and separately communicate remotely, such as in a patient home-based system to telecommunicate to clinic or hospital; or 5) a complete medical clinic system.

Peripherals and interfaces can include a UART (universal asynchronous receiver/transmitter) data interface and MCSI (multi-channel serial interface) voice and data wireless interface for an off-chip IEEE 802.15 (Bluetooth and low and high rate piconet, Zigbee, and personal network communications) for a wireless circuit. The Bluetooth or Zigbee wireless interface is useful for receiving from and controlling the accelerometer sensor and its associated analog circuitry and digital to analog-to-digital converter ADC in FIG. 1. In arrangements including ECG electrodes and/or a chest microphone, the analog circuitry at the taped-on sensor unit also includes couplings from such pickup elements to the Bluetooth or Zigbee short distance transceiver from the chest sensor.

An accelerometer sensor 10 and transmitter, transceiver, or transponder chip can be firmly mounted on a thin, resilient, light-weight plastic support plate with round smoothed or flanged coin-sized periphery and comfortably affixed by adhesive tape to chest. The Z-axis of the accelerometer is perpendicular to the plane defined by the plastic support. The chip may harvest power from an interrogation signal, or may have a small battery on the plastic support electrically connected to supply low power to the chip, such as an MSP430™ low power processor from Texas Instruments or other processor and a short distance wireless transmitter. Also, a wireline interface with a miniature wireline female connector can be electrically connected to a wireline interface in the chip, such as for USB (universal serial bus).

In some embodiments, accelerometer signals from all three axes are suitably processed to electronically double-integrate the acceleration to determine the location of the person wearing it and successively estimate a varying physical location from the same signal input of a signal interface as a cue to the person who is visually impaired, to caregiver, and to a family member. Since the person is likely to have been in bed overnight, the processing determines the location of the person during the day by double-integrating the acceleration starting from initial conditions of position initially at the bed location, and zero initial vector velocity. An electronic compass can be physically included into the sensor assembly for supporting location-based services by the sensor assembly. Some embodiments have a printed wiring board (PWB) for an applications processor coupled to a modem, together and a user interface if used. SDRAM and flash memory storage coupled to the system can provide tables, configuration and parameters and an operating system (OS), protected applications (PPAs and PAs), and other supervisory software. Adjusted parameter(s) are loaded into the flash memory or otherwise, and components are assembled on PWB to produce resulting system units.

Processing circuitry comprehends digital, analog and mixed signal (digital/analog) integrated circuits, ASIC circuits, PALs, PLAs, decoders, memories, and programmable and nonprogrammable processors, microcontrollers and other circuitry. Couplings and connections can be ohmic, capacitive, inductive, photonic, and direct or indirect via intervening circuits or otherwise as desirable. Flow diagrams and block diagrams are each interpretable as representing structure and/or process.

Those skilled in the art will appreciate that many other embodiments and variations are also possible within the scope of the claimed invention.

## Claims

1. A respiration monitoring device for use with a signal input, comprising:

    a signal interface to receive and convert the signal input to an electronic form for further processing, wherein the signal input is an acceleration signal; and
    an electronic circuit fed by the signal interface and comprising a smoothing filter, and operable to filter the acceleration signal in a manner to obtain a slow wander signal, to subtract from the acceleration signal the slow wander signal to obtain a heart sound signal, and to derive a respiration signal from the heart sound signal; and
    wherein the electronic circuit is further operable to subtract the respiration signal from the slow wander signal to produce a substantially non-respiration body motion signal.

2. The device of claim 1, further comprising an accelerometer for application to the chest and coupled to deliver the signal input; whereby acceleration is possible due to both non-respiratory body motion and respiration.

3. The device of claim 1, wherein the electronic circuit is operable to derive respiration rate based on the respiration signal.

4. The device of claim 3, wherein the electronic circuit further includes a nonvolatile memory that has instructions and filter coefficients for directing operations of the electronic circuit.

5. The device of claim 4, wherein the electronic circuit further includes an electronic processor operable to perform an interpolation augmenting and based on the respiration signal for representing the respiration responsive to an accelerometer.

6. The device of claim 5, further comprising an output modem circuit coupled to the electronic circuit, and operable to transmit signals representing at least some of the information generated by the electronic circuit.

7. The device of claim 1 or 5, wherein the electronic circuit is further operable, independent of the heart signal, to separate a first signal component for body motion responsive to an accelerometer above approximately 2 Hz from a second signal component for respiration responsive to the accelerometer below approximately 2 Hz.

8. The device of claim 7, further including a respiration display circuit coupled to the electronic circuit.

9. The device of claim 7, wherein the electronic circuit is further operable to monitor for an unusually high-magnitude peak in the non-respiration body motion signal and, in such case, supplying a signal representing a possible fall of a user.

10. The device of claim 7, wherein the electronic circuit is further operable to derive a first signal representing heart pulses having amplitude modulation, and further to generate a second signal that varies responsive to the amplitude modulation to represent respiration.

11. The device of claim 10, wherein the electronic circuit is further operable to process one or more of the respiration signal, non-respiration body motion signal and heart signal in order to detect coughing.

12. The device of claim 10, wherein the electronic circuit is further operable to derive walking gait from the non-respiration body motion signal.

13. The device of claim 10, wherein the electronic circuit is further operable to successively estimate a varying physical location from the same signal input of the signal interface.

14. The device of claim 1, wherein the electronic circuit further includes an electronic processor operable to perform an interpolation augmenting and based on the respiration signal for representing the respiration responsive to an accelerometer.


**Patentansprüche**

1. Atmungsüberwachungsvorrichtung zur Verwendung mit einem Eingangssignal, Folgendes umfassend:

   eine Signalschnittstelle zum Empfangen und Umwandeln des Eingangssignals in eine elektronische Form zur weiteren Verarbeitung, wobei das Eingangssignal ein Beschleunigungssignal ist; und
   eine elektronische Schaltung, die von der Signalschnittstelle gespeist wird und einen Glättungsfilter umfasst und betriebsfähig ist, um das Beschleunigungssignal so zu filtern, dass ein langsames Wandersignal erhalten wird, um von dem Beschleunigungssignal das langsame Wandersignal zu subtrahieren, um ein Herztonsignal zu erhalten, und um aus dem Herztonsignal ein Atmungssignal abzuleiten; und
   wobei die elektronische Schaltung ferner betriebsfähig ist, das Atmungssignal von dem langsamen Wandersignal zu subtrahieren, um ein im Wesentlichen Nicht-Atmungs-Körperbewegungssignal zu erzeugen.

2. Vorrichtung nach Anspruch 1, die ferner einen Beschleunigungsmesser zur Anbringung an der Brust umfasst, der so gekoppelt ist, dass er das Eingangssignal liefert; wobei eine Beschleunigung sowohl durch nicht-respiratorische Körperbewegungen als auch durch die Atmung möglich ist.

3. Vorrichtung nach Anspruch 1, wobei die elektronische Schaltung betriebsfähig ist, die Atemfrequenz auf der Grundlage des Atmungssignals abzuleiten.

4. Vorrichtung nach Anspruch 3, wobei die elektronische Schaltung ferner einen nichtflüchtigen Speicher enthält, der Anweisungen und Filterkoeffizienten zur Steuerung von Operationen der elektronischen Schaltung enthält.

5. Vorrichtung nach Anspruch 4, wobei die elektronische Schaltung ferner einen elektronischen Prozessor enthält, der betriebsfähig ist, eine Interpolation durchzuführen, die das Atmungssignal ergänzt und darauf basiert, um die Atmung in Reaktion auf einen Beschleunigungsmesser darzustellen.

6. Vorrichtung nach Anspruch 5, die ferner eine Ausgangsmodemschaltung umfasst, die mit der elektronischen Schaltung gekoppelt ist und betriebsfähig ist, um Signale zu übertragen, die zumindest einen Teil der von der elektronischen Schaltung erzeugten Informationen darstellen.

**EP 4 154 805 B1**

7. Vorrichtung nach Anspruch 1 oder 5, wobei die elektronische Schaltung ferner betriebsfähig ist, um unabhängig vom Herzsignal eine erste Signalkomponente für Körperbewegungen in Reaktion auf einen Beschleunigungsmesser oberhalb von etwa 2 Hz von einer zweiten Signalkomponente für die Atmung in Reaktion auf den Beschleunigungsmesser unterhalb von etwa 2 Hz zu trennen.

8. Vorrichtung nach Anspruch 7, die außerdem eine mit der elektronischen Schaltung gekoppelte Atmungsanzeigeschaltung enthält.

9. Vorrichtung nach Anspruch 7, wobei die elektronische Schaltung ferner betriebsfähig ist, eine ungewöhnlich hohe Spitze in dem Nicht-Atmungs-Körperbewegungssignal zu überwachen und in einem solchen Fall ein Signal zu liefern, das einen möglichen Sturz eines Benutzers darstellt.

10. Vorrichtung nach Anspruch 7, bei der die elektronische Schaltung ferner betriebsfähig ist, ein erstes Signal abzuleiten, das Herzimpulse mit Amplitudenmodulation darstellt, und ferner ein zweites Signal zu erzeugen, das sich in Abhängigkeit von der Amplitudenmodulation ändert, um die Atmung darzustellen.

11. Vorrichtung nach Anspruch 10, wobei die elektronische Schaltung ferner betriebsfähig ist, eines oder mehrere der Atmungssignale, der Nicht-Atmungs-Körperbewegungssignale und der Herzsignale zu verarbeiten, um Husten zu erkennen.

12. Vorrichtung nach Anspruch 10, wobei die elektronische Schaltung ferner betriebsfähig ist, aus dem Nicht-Atmungs-Körperbewegungssignal die Gangart abzuleiten.

13. Vorrichtung nach Anspruch 10, wobei die elektronische Schaltung ferner betriebsfähig ist, um aus demselben Signaleingang der Signalschnittstelle sukzessive einen sich ändernden physikalischen Ort zu schätzen.

14. Vorrichtung nach Anspruch 1, wobei die elektronische Schaltung ferner einen elektronischen Prozessor enthält, der betriebsfähig ist, eine Interpolation durchzuführen, die das Atmungssignal ergänzt und darauf basiert, um die Atmung in Reaktion auf einen Beschleunigungsmesser darzustellen.


**Revendications**

1. Dispositif de surveillance de la respiration pour une utilisation avec une entrée de signal, comprenant :

   une interface de signal pour recevoir l'entrée de signal et pour la convertir selon une forme électronique pour un traitement ultérieur, dans lequel l'entrée de signal est un signal d'accélération ; et
   un circuit électronique alimenté par l'interface de signal et comprenant un filtre de lissage, et pouvant être rendu opérationnel pour filtrer le signal d'accélération de manière à obtenir un signal à déviation lente, pour soustraire du signal d'accélération le signal à déviation lente pour obtenir un signal de bruit du coeur et pour dériver un signal de respiration à partir du signal de bruit du coeur ; et
   dans lequel le circuit électronique peut en outre être rendu opérationnel pour soustraire le signal de respiration du signal à déviation lente pour produire un signal de mouvement du corps sensiblement en non respiration.

2. Dispositif selon la revendication 1, comprenant en outre un accéléromètre pour une application sur la poitrine et couplé pour délivrer l'entrée de signal ; d'où il résulte qu'une accélération est possible du fait à la fois du mouvement du corps en non respiration et de la respiration.

3. Dispositif selon la revendication 1, dans lequel le circuit électronique peut être rendu opérationnel pour dériver une fréquence de respiration sur la base du signal de respiration.

4. Dispositif selon la revendication 3, dans lequel le circuit électronique inclut en outre une mémoire non volatile qui comporte des instructions et des coefficients de filtre pour diriger des opérations du circuit électronique.

5. Dispositif selon la revendication 4, dans lequel le circuit électronique inclut en outre un processeur électronique pouvant être rendu opérationnel pour réaliser une augmentation par interpolation et sur la base du signal de respiration pour représenter la respiration en réponse à un accéléromètre.

**6.** Dispositif selon la revendication 5, comprenant en outre un circuit de modem de sortie couplé au circuit électronique et pouvant être rendu opérationnel pour transmettre des signaux représentant au moins une certaine part de l'information générée par le circuit électronique.

**7.** Dispositif selon la revendication 1 ou 5, dans lequel le circuit électronique peut en outre être rendu opérationnel, indépendamment du signal cardiaque, pour séparer une première composante de signal pour le mouvement du corps en réponse au fait qu'un accéléromètre est au-delà d'approximativement 2 Hz vis-à-vis d'une seconde composante de signal pour la respiration en réponse au fait que l'accéléromètre est en-deçà d'approximativement 2 Hz.

**8.** Dispositif selon la revendication 7, incluant en outre un circuit d'affichage de respiration couplé au circuit électronique.

**9.** Dispositif selon la revendication 7, dans lequel le circuit électronique peut en outre être rendu opérationnel pour exercer une surveillance quant à une crête d'amplitude élevée de façon inhabituelle dans le signal de mouvement du corps en non respiration et, dans un tel cas, pour fournir un signal représentant une chute possible d'un utilisateur.

**10.** Dispositif selon la revendication 7, dans lequel le circuit électronique peut en outre être rendu opérationnel pour dériver un premier signal représentant des impulsions cardiaques présentant une modulation d'amplitude, et en outre pour générer un second signal qui varie en réponse à la modulation d'amplitude pour représenter la respiration.

**11.** Dispositif selon la revendication 10, dans lequel le circuit électronique peut en outre être rendu opérationnel pour traiter un signal ou plusieurs signaux parmi le signal de respiration, le signal de mouvement du corps en non respiration et le signal cardiaque afin de détecter une toux.

**12.** Dispositif selon la revendication 10, dans lequel le circuit électronique peut en outre être rendu opérationnel pour dériver une action de marche à partir du signal de mouvement du corps en non respiration.

**13.** Dispositif selon la revendication 10, dans lequel le circuit électronique peut en outre être rendu opérationnel pour estimer successivement une localisation physique variable à partir de la même entrée de signal de l'interface de signal.

**14.** Dispositif selon la revendication 1, dans lequel le circuit électronique inclut en outre un processeur électronique pouvant être rendu opérationnel pour réaliser une augmentation par interpolation et sur la base du signal de respiration pour représenter la respiration en réponse à un accéléromètre.

*FIG. 1*

LPF/ADC INPUT (Z-AXIS)

110 → LOW PASS FILTER THE INPUT (FILTER CUTOFF = 50 Hz)

120 → DECIMATE BY 10      PHASE 1

x(i)

130 → SAVITZKY-GOLAY POLYNOMIAL FILTERING (OR OTHER MOTION-EXTRACTION)

g(i) → SLOW MOTION WANDER

140 → SUBTRACT SAVITZKY-GOLAY OUTPUT FROM THE LOW-PASS FILTER OUTPUT TO OBTAIN HEART SOUNDS, S1 AND S2

PHASE 2

r(i) → HEART SOUND SIGNAL

150 → ENVELOPE-BASED NOISE REJECTION

R(i)

160 → FOLDED CORRELATION ON HEART SOUND SIGNAL

$f_c(i)$

170 → PEAK DETECTION

180 → COUNT/GENERATE HEART RATE SIGNAL

*FIG. 2*

RAW SIGNAL

SAVITZKY-GOLAY POLYNOMIAL FIT

LPF AT 2 Hz

MOTION

FOLDED CORRELATION, PEAK DETECTION

RESPIRATION (S1-S1 INTERVAL)

HEART-RATE

*FIG. 3*

310 — HEART SOUND SIGNAL
(FROM PHASE 2)

320 — PERFORM FOLDED
CORRELATION ON
HEART SOUND SIGNAL

330 — PERFORM PEAK DETECTION
AND OBTAIN S1 AND S2
PEAK LOCATIONS

340 — COMPUTE S1-TO-S1 OR
S2-TO-S2 INTERVAL

350 — INTERPOLATE THE INTERVAL
VALUES TO OBTAIN ESTIMATE
OF RESPIRATION SIGNAL

*FIG. 4*

INPUT

380 — LOW PASS FILTER THE INPUT
(FILTER CUTOFF = 2 Hz)

390 — RESPIRATION SIGNAL

*FIG. 5*

410 — HEART SOUND SIGNAL
(FROM PHASE 2)

420 — PERFORM FOLDED
CORRELATION ON
HEART SOUND SIGNAL

430 — PERFORM PEAK DETECTION
AND OBTAIN S1/S2 PEAKS

440 — OBTAIN S1 OR S2
PEAK AMPLITUDES

450 — INTERPOLATE THE AMPLITUDE
(OR POWER) VALUES TO
OBTAIN ESTIMATE OF
RESPIRATION SIGNAL

*FIG. 6*

*FIG. 7*

EP 4 154 805 B1

**EP 4 154 805 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004111025 A
- US 2008275349 A
- US 2009105556 A
- US 2005240087 A

**Non-patent literature cited in the description**

- Mapping the Human Body for Vibrations using an Accelerometer. **DAVID BARBAROSA RENDON et al.** 2009 ANNUAL INTERNATIONAL CONFERENCE. THE IEEE ENGINEERING IN MEDICINE AND BIOLOGY SOCIETY